# EUROPEAN PATENT APPLICATION

(11) **EP 4 112 059 A1**
(43) Date of publication of application: **04.01.2023**
(21) Application number: 20921268.7
(22) Date of filing: 27.02.2020
(51) Int. Cl.: A61K 31/715, A61K 9/14, A61P 7/04

(54) **MULTI-USE HEMOSTATIC COMPOSITION AND METHOD FOR PRODUCING SAME**

(30) Priority: 25.02.2020 KR 20200022652
(71) Applicant: Theracion Biomedical Co. Ltd., Jungwon-gu Seongnam-si, Gyeonggi-do 13201 (KR)
(72) Inventor: KIM, Eun Jin, Seongnam-si Gyeonggi-do 13587 (KR); KIM, Sun Jong, Seongnam-si Gyeonggi-do 13208 (KR); KIM, Byung Nam, Seongnam-si Gyeonggi-do 13228 (KR)
(74) Representative: Klunker IP Patentanwälte PartG mbB
(86) International application number: PCT/KR2020/002840
(87) International publication number: WO 2021/172624

(57) **Abstract**

The present invention relates to a multi-use hemostatic composition and a method for producing the same, and more particularly, to a technique relating to a multi-use a hemostatic agent in which a first component having a modified anionized substituent and a second component that is a polymer having adhesiveness are included to provide improved hemostatic ability and biocompatibility so as to have applicability into surgery, laparoscopy, and various surgical procedures.

## Description

### TECHNICAL FIELD

The present invention relates to a multi-use hemostatic composition and a method for producing the same, and more particularly, to a technique relating to a multi-use a hemostatic agent in which a first component having a modified anionized substituent and a second component that is a polymer having adhesiveness are included to provide improved hemostatic ability and biocompatibility so as to have applicability into surgery, laparoscopy, and various surgical procedures.

### BACKGROUND ART

Bleeding means drainage of blood out of blood vessels. The blood vessels that make up the whole body circulate the blood in the blood vessels throughout the body by the pressure of the heart. When a wound occurs in some blood vessels, a space through which the pressure escapes is generated and blood leaks out of the wound. Bleeding, in which blood is drained out of the blood vessels, may occur in daily life injuries and medical practices such as surgery.

Hemostasis is a process to stop bleeding and refers to maintaining blood in damaged blood vessels. At the time of hemostasis, it is of utmost importance to quickly stop bleeding from a bleeding site by suturing or compressing a wound area with a hemostatic agent, bandage, or dressing. In particular, in recent years, the demand for hemostatic agents having excellent hemostatic ability while having excellent human-friendly biocompatibility is increasing. In order to respond to this, hemostatic agents using non-toxic natural polymers as the main material are being developed to meet improved hemostatic effect and various demands of the markets.

For example, Korean Patent Publication No. 10-2014-0074993 discloses a hemostatic composition including a biocompatible polymer in particulate form suitable for use in hemostasis and one hydrophilic polymer component including reactive groups. More specifically, Korean Patent Publication No. 10-2014-0074993 discloses that the combination of one hydrophilic polymer component with the biocompatible polymer in particulate form has improved hemostasis properties and can provide improved tissue adherence.

Korean Patent Registration No. 10-1301276 relates to an adhesive hydrogel composition including chitosan to which a catechol group is bonded and pluronic in which a thiol group is bonded to a terminal. More specifically, Korean Patent Registration No. 10-1301276 discloses an adhesive composition that can be used as a bioadhesive agent because it is temperature-sensitive and has excellent hemostatic effect while providing safety inside and outside the body, and a medical adhesive, an anti-adhesion agent, and a surface adsorption inhibitor.

Korean Patent Publication No. 10-2017-0060054 discloses a biocompatible hemostatic product or a tissue sealant. More specifically, Korean Patent Publication No. 10-2017-0060054 discloses that polyoxyethylene granules are included, the viscosity average molecular weight of the polyoxyethylene is 100,000 Daltons to 7,000,000 Daltons, the particle diameter of the polyoxyethylene granules is 0.5 µm to 2,000 µm, and the absorption ratio is 1 to 500. Accordingly, it is characterized in that it can provide effects such as hemostasis, blocking prevention, infection prevention, promotion of tissue union and wound sealing on the bleeding wound surfaces of tissue organs and body cavities.

As described above, various research and development on hemostatic agents with excellent biocompatibility are being actively conducted. As one of such research, the present invention has been completed to provide a multi-use hemostatic agent that can improve hemostatic ability and biocompatibility through a method of surface-modifying a biopolymer that provides a hemostatic effect, and can be applied to surgery, laparoscopy, and various surgical procedures.

### [Citation List]

(Patent Literature 1) Korean Patent Publication No. 10-2014-0074993 (2014.06.18)
(Patent Literature 2) Korean Patent Registration No. 10-1301276 (2013.08.29)
(Patent Literature 3) Korean Patent Publication No. 10-2017-0060054 (2017.05.31)

### DESCRIPTION OF EMBODIMENTS

### TECHNICAL PROBLEM

An object of the present invention is to solve the problems of the related art and the technical problems as described above.

An object of the present invention is to provide an improved hemostatic action while providing excellent blood absorption and biocompatibility, including a component having a modified anionized substituent and an adhesive polymer.

An object of the present invention is to provide a multi-use hemostatic agent applicable to surgery, laparoscopy, and various surgical procedures.

### SOLUTION TO PROBLEM

According to an embodiment of the present invention, there is provided a multi-use hemostatic composition including: a first component having a modified anionized substituent; and a second component including a polymer having adhesiveness.

According to an embodiment of the present invention, there is provided a method for producing a multi-use hemostatic composition, the method including: (a) adding a first component having an anionized substituent to a solution and stirring to prepare a first component having a modified anionized substituent; (b) separating a supernatant by centrifugation and obtaining a powder of the first component having the modified anionized substituent; (c) washing and drying the powder; and (d) mixing the powder of the first component having the anionized substituent modified by the drying with a second component including a polymer having adhesiveness.

### ADVANTAGEOUS EFFECTS OF INVENTION

A hemostatic agent according to the present invention has excellent blood and biocompatibility and maximizes blood absorption ability to promote hemostatic coagulation, thereby providing improved hemostasis.

The present invention may provide a multi-use hemostatic agent applicable to surgery, laparoscopy, and various surgical procedures.

A hemostatic agent according to the present invention may be provided in powder form, thereby providing ease of storage and handling.

### BRIEF DESCRIPTION OF DRAWINGS

FIG. 1 shows a scanning electron microscope (SEM) photograph of a multi-use hemostatic composition produced according to an embodiment of the present invention.
FIG. 2 shows a result of cytotoxicity evaluation according to Experimental Example 1 of the present invention.
FIG. 3 shows a result of biodegradation evaluation according to Experimental Example 2 of the present invention.
FIG. 4 shows a result of an in vitro hemostatic ability test according to Experimental Example 3 of the present invention.
FIG. 5 shows photographs and descriptions according to the in vivo test sequence of the present invention.
FIG. 6 shows a result of an in vitro hemostatic ability test according to Experimental Example 4 of the present invention.

### BEST MODE

Reference is made to the accompanying drawing which shows, by way of illustration, specific embodiments in which the present invention may be practiced. The embodiments will be described in detail in such a manner that the present invention can be carried out by those of ordinary skill in the art. It should be understood that various embodiments of the present invention are different from each other, but need not be mutually exclusive. For example, certain shapes, structures, and features described herein may be implemented in other embodiments without departing from the spirit and scope of the present invention in connection with one embodiment. In addition, it will be understood that the locations or arrangement of individual components in the disclosed embodiments can be changed without departing from the spirit and scope of the present invention. Therefore, the following detailed description is not to be taken in a limiting sense, and the scope of the present invention is to be limited only by the appended claims and the entire scope of equivalents thereof, if properly explained. Like reference numerals in the drawing refer to the same or similar functions throughout the various aspects.

Hereinafter, preferred embodiments of the present invention will be described in detail with reference to the accompanying drawing, so that those of ordinary skill in the art can easily carry out the present invention.

According to an embodiment of the present invention, a multi-use hemostatic composition and a method for producing the same are provided.

According to an embodiment of the present invention, there is provided a multi-use hemostatic composition including: a first component having a modified anionized substituent; and a second component including a polymer having adhesiveness.

According to an embodiment of the present invention, the first component having the modified anionized substituent may include a bio-based polymer including an anionized substituent, for example, at least one selected from carboxymethyl starch, carboxyethyl starch, carboxymethylcellulose, carboxyethyl cellulose, carboxymethyl alginate, polyacrylic acid-starch graft copolymer, starch-2-hydroxypropylcitrate, starch-2-hydroxypropylphosphate, and starch-2-hydroxypropylsulfate. Therefore, the first component having the modified anionic substituent may provide biocompatibility, biodegradability, and hydrophilicity, and may provide improved hemostatic ability, compared to the related art.

According to an embodiment of the present invention, the modification may be performed by including at least one selected from calcium chloride, calcium carbonate, calcium citrate, calcium gluconate, calcium hydroxide, and calcium oxalate. Preferably, calcium chloride may be provided so that the anionized substituent can be modified, and when the anionized substituent is modified by calcium, hydrophilicity may be improved so that fluid absorption and expandability can be improved. In addition, since calcium ions can play a helpful role in hemostasis, hemostatic ability can be remarkably improved and can help improve blood coagulation. This can be confirmed through the results of the experimental examples of Examples to be described below.

According to an embodiment of the present invention, the second component including the polymer having the adhesiveness may include at least one selected from alginate, chitosan, chitosan derivatives, guar gum, xanthan gum, pullulan, carrageenan, hyaluronic acid, polyethylene glycol, polyacrylic acid, polyvinylpyrrolidone, cellulose, and cellulose derivatives.

For example, alginate may be provided as the second component including the polymer having the adhesiveness, and alginate refers to a natural polymer aggregate including polysaccharides called alginic acid. It is a compound formed by randomly combining unbranched 1,4-linked mannuronic acid (β-d-mannuronic acid) and α-L-gluconic acid. The physical properties of alginate gel change according to the type of seaweed from which an M/G ratio, that is, a composition ratio of M and G chains, is extracted. As the G content of alginate is larger, the tendency to form a hard porous gel and maintain a gel state for a long time is stronger. During cross-linking with divalent cations, excessive swelling and shrinkage do not occur, thereby maintaining an original shape. On the other hand, when the content of M is increased, a flexible and less porous gel is formed. This gelation of alginate is due to formation of a three-dimensional structure of an egg box model having a V-shaped hole when G reacts with divalent cations. Therefore, the M/G ratio may be adjusted as necessary. In addition, although the alginate is insoluble, the alginate may be provided in the form of alginic acid salt, thereby exhibiting water solubility. For example, sodium alginate may be provided. In the case of the alginic acid, the molecule thereof basically has a carboxyl group (-COOH) and has a property of binding to a cation using an unshared pair of electrons of an anion after an acid-base reaction. Therefore, sodium alginate is a combination of alginate molecules with sodium cations (Na+). In this case, sodium alginate has a very flexible polymer chain structure. Accordingly, it is possible to provide adhesiveness together with biodegradability and to provide high swelling properties when in contact with moisture.

As another example, chitosan may be provided as the second component including the polymer having the adhesiveness. Chitosan is soluble in an organic acid aqueous solution and has high viscosity, which is different depending on molecular weight, degree of deacetylation, ionic strength, pH, etc. In addition, chitosan has excellent biocompatibility and has excellent adsorption properties by forming chelates with metal ions. Since chitosan absorbs body fluids or exudate, a hemostatic effect can be efficiently provided at a bleeding site. In particular, chitosan does not significantly affect the formation of the chelate even when ions of magnesium, copper, and potassium exist in a large amount. In addition, chitosan is a natural polymer that is less likely to be depleted, exhibits excellent biocompatibility in tissues or in the body, has little toxicity, and is easily biodegradable.

Furthermore, the polymer providing the adhesiveness is not limited to alginate and chitosan described above. As materials that can provide serve a similar role, chitosan derivatives, guar gum, xanthan gum, pullulan, carrageenan, hyaluronic acid, polyethylene glycol, polyacrylic acid, polyvinylpyrrolidone, cellulose, and cellulose derivatives may be provided, and the present invention is not limited thereto.

According to an embodiment of the present invention, a content ratio of the first component to the second component may be 99 to 90:1 to 10. In the above range, it is possible to provide an effect of having adhesiveness together with blood absorption ability.

According to an embodiment of the present invention, the first component and the second component are in powder form. Accordingly, excellent blood absorption ability can be expected, and ease of use, storage and handling can be provided.

According to an embodiment of the present invention, the powder form of the first component may be granulated with particles having a diameter of 1 to 500 µm. Accordingly, the powder of the first component has a porous structure so that wettability is improved, and may provide an effect of increasing a dispersion and sedimentation rate in water.

According to an embodiment of the present invention, the powder of the second component may have a diameter of 1 to 500 µm. The powder diameter can provide an appropriate specific surface area within the above range, thereby providing excellent hemostatic ability and blood absorption.

On the other hand, there is provided a method for producing a multi-use hemostatic composition according to the present invention. Hereinafter, the same description as the multi-use hemostatic composition describe above may be applied, and redundant descriptions thereof will be omitted.

According to an embodiment of the present invention, there is provided a method for producing a multi-use hemostatic composition, the method including: (a) adding a first component having an anionized substituent to a solution and stirring to prepare a first component having a modified anionized substituent; (b) separating a supernatant by centrifugation and obtaining a powder of the first component having the modified anionized substituent; (c) washing and drying the powder; and (d) mixing the powder of the first component having the anionized substituent modified by the drying with a second component including a polymer having adhesiveness.

According to an embodiment of the present invention, the method may include (a) adding a first component having an anionized substituent to a solution and stirring to prepare a first component having a modified anionized substituent. In this case, the solution may include at least one selected from calcium chloride, calcium carbonate, calcium citrate, calcium gluconate, calcium hydroxide, and calcium oxalate in an aqueous solution. For example, calcium ions may be provided by dissolving calcium chloride in an aqueous solution, and the surface of the first component having the anionized substituent may be modified by the provided calcium ions. When the anionized substituent is modified by calcium, hydrophilicity may be improved and fluid absorption and expandability may also be improved. Accordingly, it is possible to remarkably improve the hemostatic ability and provide a role of helping the blood coagulation mechanism.

According to an embodiment of the present invention, the solution in the step (a) may have a concentration of 5 to 75%. When the concentration is less than 5%, the hemostatic ability is reduced due to lack of calcium ions, and when the concentration is greater than 75%, CaCl₂ is in excess, and thus, there is a problem in that it is present in a saturated solid state in a solution phase. In addition, when calcium ions are present in excess, the activity of thrombin in the blood is reduced to cause an adverse effect on hemostasis. Therefore, the solution may be preferably provided in a concentration of 5 to 75%.

According to an embodiment of the present invention, the first component having the anionized substituent in the step (a) may include at least one selected from carboxymethyl starch, carboxyethyl starch, carboxymethylcellulose, carboxyethyl cellulose, carboxymethyl alginate, polyacrylic acid-starch graft copolymer, starch-2-hydroxypropylcitrate, starch-2-hydroxypropylphosphate, and starch-2-hydroxypropylsulfate. Accordingly, it is possible to provide biocompatibility, biodegradability, and hydrophilicity and provide improved hemostatic ability, compared to the related art.

According to an embodiment of the present invention, a stirring time in the step (a) may be 30 minutes to 12 hours. When the stirring time is less than 30 minutes, the reaction does not proceed sufficiently, and when the stirring time is greater than 12 hours, the stirring time is longer than necessary. Therefore, the above range is preferable.

According to an embodiment of the present invention, the method may include (b) separating a supernatant by centrifugation and obtaining a powder of the first component having the modified anionized substituent. In this case, the obtained powder of the first component may be granulated with particles having a diameter of 1 to 500 µm.

After the step (a), in order to fractionate the first component dissolved in the solution, the supernatant may be separated using centrifugation to obtain the powder of the first component having the modified anionized substituent. In this case, a reduced pressure filter may be used in the process of separating the supernatant. The reduced pressure filter refers to a device that performs filtering using a suction force for a filtrate generated by reducing pressure inside a flask below a filter. It is possible to separate and obtain the first component while removing the supernatant with the reduced pressure filter. By providing the reduced pressure filter, a large amount of material can be filtered within a short time and the efficiency of filtration can also be improved.

According to an embodiment of the present invention, the method may include (c) washing and drying the powder. The washing is preferably performed using ethanol 2 to 5 times at room temperature to completely remove foreign substances. In addition, the number of times can be adjusted as necessary. The drying may be at least one selected from freeze-drying and heat-drying. Preferably, heat-drying may be provided.

For example, in the case of the freeze-drying, a preferred method is to rapidly freeze micro-beads at 0°C to -70°C to remove a solvent under vacuum conditions. Preferably, the freeze-drying may be provided at -30 to -40°C for 1 to 48 hours, and preferably 2 to 24 hours.

The heat-drying may be provided at 70 to 100°C. Preferably, the drying may be performed in an oven of 75 to 85°C. In this case, the drying time may be 10 minutes to 1 hour, and preferably 10 minutes to 30 minutes.

According to an embodiment component the present invention, the method may include (d) mixing the powder of the first component having the anionized substituent modified by the drying with a second component including a polymer having adhesiveness. Accordingly, a multi-use hemostatic composition is finally produced.

According to an embodiment of the present invention, the mixing in the step (d) may include mixing the powder of the first component with the second component including the polymer having the adhesiveness in a ratio of 99 to 90:1 to 10. In the above range, it is possible to provide an effect of having adhesiveness together with blood absorption ability.

In addition, the powder of the first component provided in this case may be granulated with particles having a diameter of 1 to 500 µm, and the powder of the second component may have a diameter of 1 to 500 µm. The powder diameter can provide a desirable specific surface area within the above range, thereby providing excellent hemostatic ability, blood coagulation, and blood absorption effects.

Hereinafter, the present invention will be described by way of examples, and the present invention is not limited thereto.

### <Example 1>

Carboxymethyl starch was added to an aqueous solution of calcium chloride (CaCl₂) and stirred for 1 hour. After the stirring, a supernatant was removed using a centrifuge and a powder was obtained and filtered under reduced pressure. At the time of the filtering under reduced pressure, the powder was washed 5 times with ethanol, and the washed powder was dried in a drying oven to obtain a first component.

A second component including an adhesive polymer was mixed with the first component to produce a multi-use hemostatic composition. In this case, a mixing ratio of the first component to the second component was 99:1.

### <Comparative Example 1>

A commercial product Arista-AH was prepared.

### <Comparative Example 2>

A commercial product Perclot was prepared.

### <Control group>

If necessary, Control (control group) was conducted to confirm an experimental effect without prescribing hemostatic agents.

### < Experimental Example 1: Cytotoxicity evaluation>

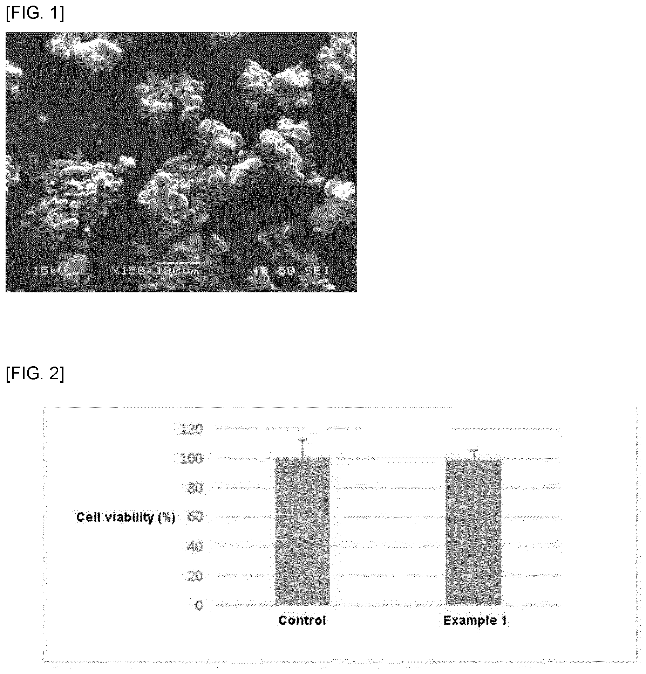

In order to evaluate the cytotoxicity of the multi-use hemostatic agents according to Examples and Comparative Examples, the cytotoxicity test was conducted in accordance with ISO 10993-5: Biological evaluation of medical devices, Part 5: Tests for in vitro cytotoxicity and the Common Standards for Biological Safety of Medical Devices provided by the Ministry of Food and Drug Safety. It was conducted to evaluate whether the test substance caused cytotoxicity to L-929 (mouse fibroblast) cells in a cell culture environment. The results thereof are shown in FIG. 2.

### < Experimental Example 2: Biodegradation evaluation>

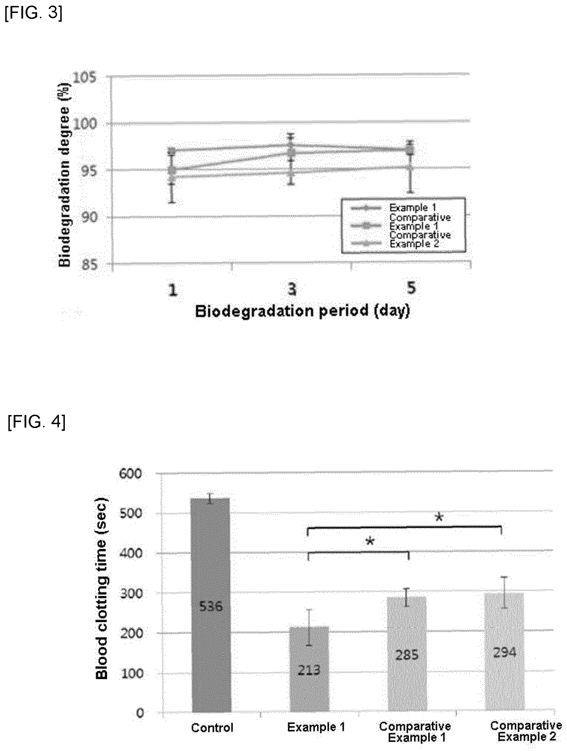

In order to evaluate the biodegradability of the multi-use hemostatic agents according to Examples and Comparative Examples, a biodegradation experiment was performed. For the test, a biodegradable solution was prepared by dissolving amylase present in a body in a simulated body fluid (SBF) solution and was injected into a sample. Thereafter, the biodegradation evaluation was performed by observing the change in weight over time. The results thereof are shown in FIG. 3 and Table 1.

**[Table 1]**

| Experimental group | Biodegradation period (day) | | |
|---|---|---|---|
| | 1 | 2 | 3 |
| Example 1 | 97.06± 0.29 | 97.57± 0.71 | 97.04± 0.14 |
| Comparative Example 1 | 95.00± 1.53 | 96.71± 2.02 | 96.98± 0.58 |
| Comparative Example 2 | 94.22± 2.71 | 94.62± 1.25 | 95.17± 2.75 |

### < Experimental Example 3: In vitro hemostatic ability test>

In order to evaluate the hemostatic ability of the multi-use hemostatic agents according to Examples and Comparative Examples, an in vitro experiment was performed in the following order in accordance with a Lee White method. The results thereof are shown in FIG. 4.
① 3 ml of blood was added to a glass vial.
② The glass vial was placed in a 37°C constant temperature water bath.
③ The hemostatic agent sample according to Example or Comparative Example was added to the glass vial and then shaken lightly.
④ A 0.025M calcium chloride (CaCl₂) solution was dropped onto 0.3 ml blood and was mixed by shaking the glass vial.
(5) A blood clotting time was measured.

### < Experimental Example 4: In vivo hemostatic ability test>

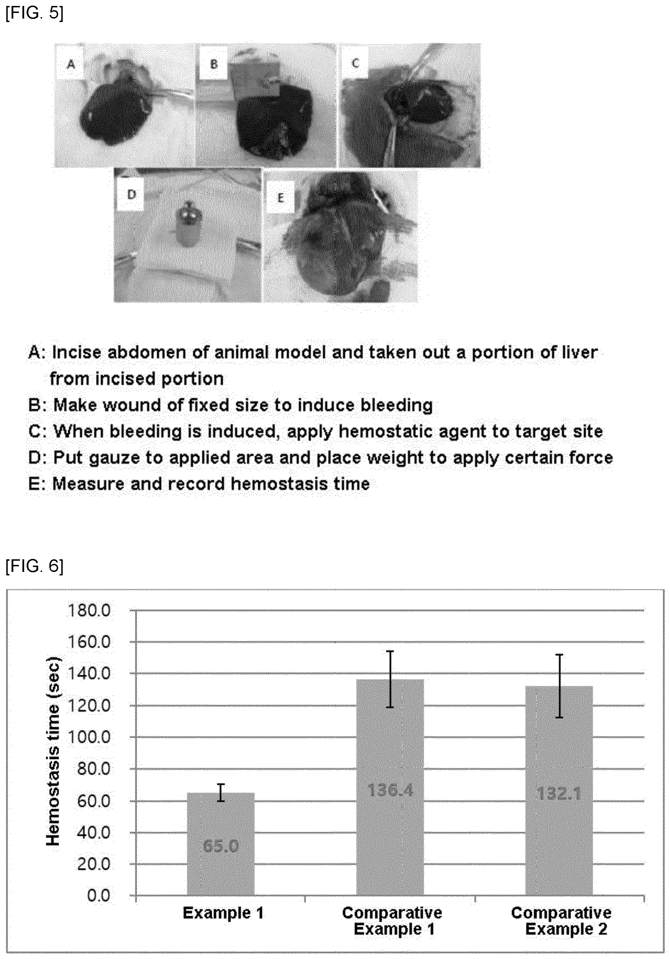

In order to evaluate the hemostatic ability of the multi-use hemostatic agents according to Examples and Comparative Examples, an in vivo experiment was performed in the following order. For reference, photographs for experimental sequence and description thereof are shown in FIG. 5. In addition, the in vivo experiment results according to Experimental Example 4 are shown in FIG. 6 and Table 2.
○,¹ A rat's liver was taken out and an experiment was prepared. As test animals, 11 8-week-old rats were provided.
○,² A scalpel was used to cut 1×1cm in a + shape and a bleeding site was blocked with gauze.
○,³ After the gauze was removed, 0.1 g of the hemostatic agent sample according to Example or Comparative Example was applied to the bleeding site.
○,⁴ After the sample was introduced, the gauze is placed on top of the sample, and the same pressure was maintained using a 50 g weight.
○,⁵ After the sample was pressed with the gauze for 1 minute, hemostasis was checked every 15 seconds.
○,⁶ After hemostasis, the sample was rinsed with saline and rebleeding was checked.

**[Table 2]**

| Number of measurements | Hemostasis time (sec) | | |
|---|---|---|---|
| | Example 1 | Comparative Example 1 | Com parative Example 2 |
| 1 | 60 | 160 | 145 |
| 2 | 60 | 150 | 135 |
| 3 | 65 | 135 | 110 |
| 4 | 60 | 135 | 100 |
| 5 | 70 | 150 | 165 |
| 6 | 75 | 120 | 135 |
| 7 | 65 | 105 | 135 |
| Average | 65.0 | 136.4 | 132.1 |
| Standard deviation | 5.3 | 17.6 | 19.9 |

### (Unit: seconds)

After ○,³, a blind test was performed and four portions of the livers of the rats were incised, and the hemostatic samples of Examples and Comparative Examples were changed in order.

From the experimental results, it could be confirmed through the SEM photograph of the multi-use hemostatic composition produced according to Example in FIG. 1 that powder having a certain diameter was granulated and aggregated.

Referring to FIG. 2, which is the cytotoxicity result of Experimental Example 1, in the case of Example 1, it was confirmed that cell viability was about 98%, compared to the control group (control). The control group (control) used a polystyrene cell culture dish, and cell viability was observed using a medium prepared by extracting the sample of Example 1. When the cell viability of the control group (control) was set to 100%, the numerical value expressed in FIG. 2 shows a cell viability of 98% in the environment of the medium prepared by extracting the sample of Example 1. This means that it meets the criteria for evaluating cell lysis and the biosafety test criteria for medical devices in accordance with ISO 10993-5, Determination of cytotoxicity, and means that the hemostatic agent according to Experimental Example 1 is biocompatible.

Referring to FIG. 3 and Table 1, which are the biodegradation evaluation results of Experimental Example 2, it was confirmed that the biodegradation degree of the Example was excellent during a similar biodegradation period.

From the results of FIG. 4, which is the result of the in vitro experiment of Experimental Example 3, it was confirmed that the blood clotting time was shortened in Example, compared to Comparative Example.

From the results of FIG. 6, which is the result of the in vivo experiment of Experimental Example 4, it was confirmed that the hemostasis time was significantly reduced to less than half, compared to Comparative Example. Therefore, it can be confirmed that the blood clotting time and hemostatic ability, which are essential conditions of the hemostatic agent, are very excellent.

Therefore, the multi-use hemostatic composition according to the present invention can manage bleeding by immediately inducing hemostasis. In particular, the multi-use hemostatic composition according to the present invention enables rapid surgery by securing the surgeon's field of vision during surgical procedures and can quickly stop excessive bleeding. Accordingly, it can be confirmed that the multi-use hemostatic composition according to the present invention is effective. In addition, since the multi-use hemostatic composition according to the present invention can be provided in powder form, it can be easily and conveniently applied to laparoscopy and various procedures.

Although the embodiments of the present invention have been described with reference to the accompanying drawings, those of ordinary skill in the art to which the present invention pertains will understand that the present invention may be embodied in other specific forms without changing the technical spirit or essential features thereof. Therefore, it should be understood that the embodiments described above are illustrative in all aspects and are not restrictive.

Therefore, it will be understood that the spirit of the present invention should not be limited to the above-described embodiments and the claims and all equivalent modifications fall within the scope of the present invention.

## Claims

1. A multi-use hemostatic composition comprising:
a first component having a modified anionized substituent; and
a second component including a polymer having adhesiveness.

2. The multi-use hemostatic composition of claim 1, wherein the modification is performed by including at least one selected from calcium chloride, calcium carbonate, calcium citrate, calcium gluconate, calcium hydroxide, and calcium oxalate.

3. The multi-use hemostatic composition of claim 1, wherein the first component having the anionized substituent includes at least one selected from carboxymethyl starch, carboxyethyl starch, carboxymethylcellulose, carboxyethyl cellulose, carboxymethyl alginate, polyacrylic acid-starch graft copolymer, starch-2-hydroxypropylcitrate, starch-2-hydroxypropylphosphate, and starch-2-hydroxypropylsulfate.

4. The multi-use hemostatic composition of claim 1, wherein the second component including the polymer having the adhesiveness includes at least one selected from alginate, chitosan, chitosan derivatives, guar gum, xanthan gum, pullulan, carrageenan, hyaluronic acid, polyethylene glycol, polyacrylic acid, polyvinylpyrrolidone, cellulose, and cellulose derivatives.

5. The multi-use hemostatic composition of claim 1, wherein a content ratio of the first component to the second component is 99 to 90:1 to 10.

6. The multi-use hemostatic composition of claim 1, wherein the first component and the second component are in powder form.

7. The multi-use hemostatic composition of claim 6, wherein the powder form of the first component is granulated with particles having a diameter of 1 to 500 µm.

8. The multi-use hemostatic composition of claim 6, wherein the powder form of the second component has a diameter of 1 to 500 µm.

9. A method for producing a multi-use hemostatic composition, the method comprising:
(a) adding a first component having an anionized substituent to a solution and stirring to prepare a first component having a modified anionized substituent;
(b) separating a supernatant by centrifugation and obtaining a powder of the first component having the modified anionized substituent;
(c) washing and drying the powder; and
(d) mixing the powder of the first component having the anionized substituent modified by the drying with a second component including a polymer having adhesiveness.

10. The method of claim 9, wherein the solution in the step (a) includes at least one selected from calcium chloride, calcium carbonate, calcium citrate, calcium gluconate, calcium hydroxide, and calcium oxalate.

11. The method of claim 9, wherein the solution in the step (a) has a concentration of 5 to 75%.

12. The method of claim 9, wherein the first component having the anionized substituent in the step (a) includes at least one selected from carboxymethyl starch, carboxyethyl starch, carboxymethylcellulose, carboxyethyl cellulose, carboxymethyl alginate, polyacrylic acid-starch graft copolymer, starch-2-hydroxypropylcitrate, starch-2-hydroxypropylphosphate, and starch-2-hydroxypropylsulfate.

13. The multi-use hemostatic composition of claim 9, wherein a stirring time in the step (a) is 30 minutes to 12 hours.

14. The method of claim 9, wherein the powder of the first component having the modified anionized substituent in the step (b) is granulated with particles having a diameter of 1 to 500 µm.

15. The method of claim 9, wherein the drying in the step (c) is one selected from freeze-drying and heat-drying.

16. The method of claim 9, wherein the second component including the polymer having the adhesiveness in the step (d) has a diameter of 1 to 500 µm.

17. The method of claim 9, wherein the mixing in the step (d) comprises mixing the powder of the first component with the second component including the polymer having the adhesiveness in a ratio of 99 to 90:1 to 10.
